# EUROPEAN PATENT SPECIFICATION

(11) **EP 0 358 203 B1**
(45) Date of publication and mention of the grant of the patent: **17.05.1995**
(21) Application number: 89116478.2
(22) Date of filing: 06.09.1989
(51) Int. Cl.: A61B 5/00

(54) **Fiber optic prove for measuring reflectance spectrum**
Fiberoptische Sonde zum Messen des Reflexionsspektrums
Sonde à fibre optique pour mesurer le spectre de réflectance

(30) Priority: 09.09.1988 JP 119058/88
(43) Date of publication of application: 14.03.1990
(73) Proprietor: SUMITOMO ELECTRIC INDUSTRIES, LTD., Osaka-shi, Osaka 541 (JP)
(72) Inventor: Kanda, Masahiko, 1-chome Konohana-ku Osaka (JP)
(74) Representative: Herrmann-Trentepohl, Werner, Dipl.-Ing.

(56) References cited:
- EP-A- 0 063 431
- EP-A- 0 093 927
- EP-A- 0 216 138
- DE-A- 2 061 098
- FR-A- 2 596 530

## Description

The present invention relates to a fiber optic probe for measuring reflectance spectrum. More specifically, the present invention relates to a reflected spectrum measuring probe for measuring hemodynamics and oxygen sufficiency such as volume of hemoglobin and oxygen saturation in blood near the surface of a living body and accordingly the diagnose state of organs such as liver by irradiating the surface of a living body with light and by analyzing the light transmitted through and reflected from the organism in accordance with spectrophotometry.

### Description of the Background Art

A fiber optic probe for measuring the reflectance spectrum has been used for analyzing the state of pigments in the surface layer of a living body by measuring the light irradiating and reflected from the surface of the organism. Such fiber optic probe comprises a fiber bundle including light illuminating optical fibers and light receiving optical fibers in which plurality of optical fibers formed of transparent materials such as plastic or glass are bundled in parallel, and a coverage provided on the outer periphery of the bundle. Such a fiber bundle used as a measuring probe is known from, for example, Japanese Utility Model Laying Open No. 59-113749.

The optical fiber generally comprises a core and a clad covering the core, with the diameter of the core being several 10 »m and the diameter of the clad being about 150 »m.

Japanese Utility Model Laying Open No. 59-113749 discloses a metal sleeve holder provided at the outermost periphery so as to protect the probe and to facilitate the use of the probe. However, what is disclosed is a number of common optical fibers bundled, each consisted of a core and a clad. The diameter of the clad is about 100 »m and diameter of the core is about several 10 »m, in a generally used plastic fiber or a glass fiber.

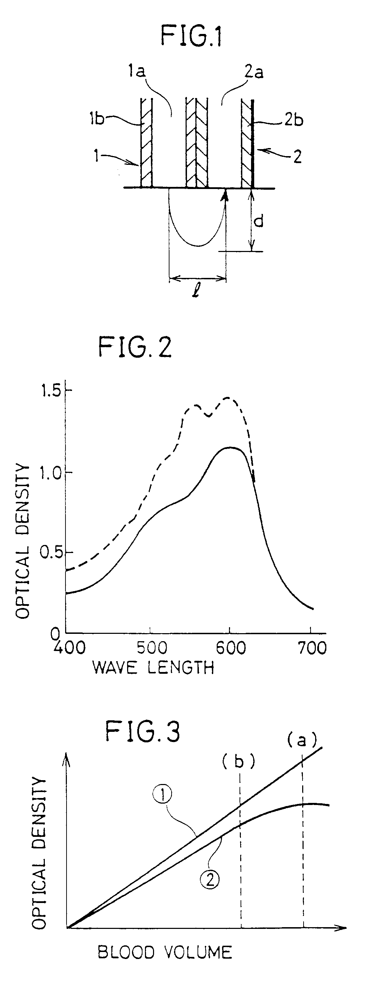

Fig. 1 is an enlarged view of a portion of a conventional probe. Fig. 2 shows a reflectance spectrum with distortion measured by the conventional probe. Fig. 3 is a graph showing the relation between the blood volume and the optical density in a sample area.

Referring to Fig. 1, a conventional probe will be described. As shown in Fig. 1, light illuminating optical fiber 1 and a light receiving optical fiber 2 are provided adjacent to each other. The light illuminating optical fiber 1 comprises a core 1a and a clad 1b, while the light receiving optical fiber 2 comprises a core 2a and a clad 2b. Each of the cores 1a and 2a has a diameter of about several 10 »m and each of the clads 1b and 2b has a diameter of about 100 »m. Therefore, the distance ℓ between the centers of the light illuminating optical fiber 1 and the light receiving optical fiber 2 is naturally about 100 »m.

Now, when a portion such as a liver containing much blood is to be measured by such a probe, the reflectance spectrum measured should be as shown by the dotted line in Fig. 2. However, actually the measured result is as shown by the solid line. The reason for this is as follows. As shown by (1) in Fig. 3, the optical density linearly increases as the blood volume increases. When the wavelength of the illuminating light becomes shorter, the effect of scattering increases, so that the increase of the optical density becomes non-linear and is distorted. Such a measuring probe is not suitable for measuring functions of organs such as liver containing much blood.

FR-A-2 596 530 discloses a fiber optic probe with the features of the preamble of claim 1.

### SUMMARY OF THE INVENTION

Therefore, an object of the present invention is to provide a reflectance spectrum measuring probe capable of measuring condition of blood and the like with the distortion in the optical density reduced as much as possible.

Briefly stated, in the present invention, a light illuminating fiber for transmitting light and a light receiving fiber for receiving the light transmitted through an organism to be returned are arranged in parallel to form a fiber bundle, and a lens is provided over the tip end surfaces of the light transmitting fiber and the light receiving fiber.

Therefore, in accordance with the present invention, the depth of incident light transmitted through the living tissue to be reflected can be made shallower, so that the measurement can be carried out with little influence of the blood volume, whereby organs such as liver containing much blood can be measured without distortion. In addition, by providing a number of lenses having different focal length to be attached and detached, various portions or blood in different organs having different blood volume can be directly measured.

Further, according to the present invention, the fiber bundle is covered with a coating layer formed of a heat shrinkable tube and the periphery of the lens is covered by an end portion of the coating layer.

Consequently, according to the present invention, the fiber bundle and the lens can be made integral.

In another aspect of the present invention, a plurality of light illuminating optical fiber cores and a plurality of light receiving optical fiber cores are arranged adjacent to each other to form a matrix in a clad, thereby providing an image fiber. Therefore, in accordance with the present invention, the distance between the centers of the light illuminating fiber core and the light receiving fiber core can be made smaller and therefore the distortion in the reflected spectrum caused by the scattering effect can be avoided, whereby either such organs having much blood as the liver can be measured by spectrophotometry with high reliability.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is vertical sectional view showing a portion of a conventional probe enlarged;
Fig. 2 shows a reflected spectrum with distortion, measured by the conventional probe;
Fig. 3 shows the relation between the blood volume and the optical density in a sample area;
Figs. 4 and 5 are vertical sectional views of one embodiment of the present invention;
Fig. 6 is a vertical sectional view showing one embodiment of the present invention during use;
Fig. 7 is a cross sectional view showing an end portion of the probe in accordance with another embodiment of the present invention; and
Fig. 8 is a perspective view showing the whole structure of another embodiment of the present invention.

### DESCRIPTION OF THE PREFERRED EMBODIMENTS

The inventor of the present invention studied the cause of the distortion in the reflectance spectrum described in relation to the prior art. He has found that the optical density, which should be increased linearly as shown by the line (1) in Fig. 3 as the volume of blood increases, does not increase linearly due to the scattering effect when the wavelength becomes shorter, as shown in (2), and that this is the cause of distortion in the reflectance spectrum. The inventors developed the present invention based on the study. More specifically, the blood volume shown in Fig. 3 is the blood volume in the sample area of the measuring probe. Namely, when the sample area is moved from the side (a) to (b) of Fig. 3, the relation between the volume of blood and the optical density corresponding to that area becomes linear, enabling measurement without distortion. The depth d of the sample area shown in Fig. 1 depends on the distance in Fig. 1, so that the depth d becomes shallower when the distance ℓ is made smaller.

The blood volume having influence to the distortion is the blood volume in the sample area of the measuring probe, which sample area depends on the distance ℓ between the light transmitting optical fiber 1 and the light receiving optical fiber 2, as is apparent from Fig. 1 showing the state of use of the conventional measuring probe. Therefore, when the distance ℓ is made smaller such that the sample area is moved from (a) to (b) in Fig. 3 showing the volume of blood, measurement without distortion becomes possible. More specifically, in the present embodiment, the distance ℓ' is smaller than ℓ and therefore the depth d' is shallower than d, whereby the distortion caused by the large blood volume can be made smaller.

One embodiment of the present invention will be described based on this understanding.

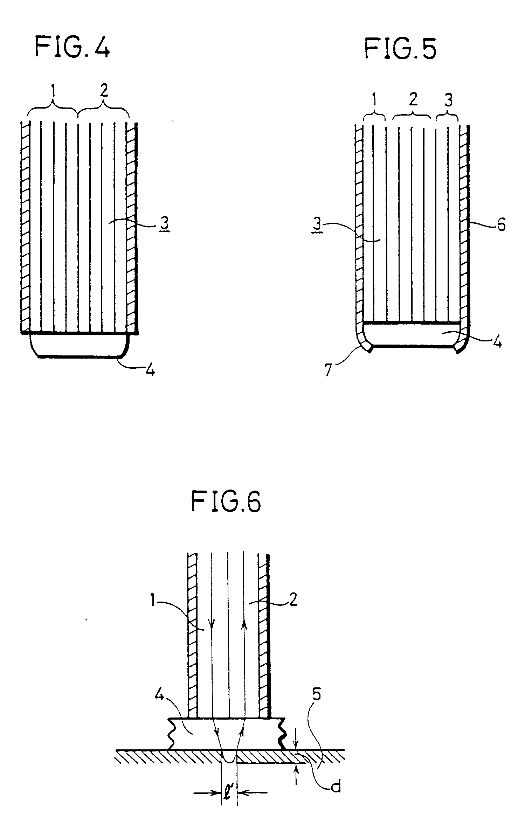

Figs. 4 and 5 are vertical sectional views of one embodiment of the present invention. Referring to Fig. 4, a light illuminating optical fiber 1 and a light receiving optical fiber 2 are put together in parallel to form a fiber bundle 3 and condenser means 4 formed of, for example, a convex lens is provided over the end surfaces of the light illuminating optical fiber 1 and the light receiving optical fiber 2 at the end portion of the fiber bundle 3. Each of the light illuminating optical fiber 1 and the light receiving optical fiber 2 comprises a bundle of a plurality of fibers formed of a transparent material such as plastic or glass. As shown in Fig. 5, the fiber bundle 3 is inserted into a heat shrinkable polyethylene tube so as to fix the bundle of the light illuminating optical fiber and the light receiving optical fiber 2. The tube is shrunk by heat to form a coating layer 6 having the thickness of 0.3 mm. The coating layer 6 extends toward the end of the fiber bundle 3 to cover the periphery of the lens 4. The end portion of the coating layer 6 is bent inward to the center of the diameter, so as to fix the lens 4 and to prevent missing of the lens.

The lens 4 is exchangeable, enabling adjustment of the degree of condensing light corresponding to the different organs containing different amounts of blood. When the lens 4 is to be exchanged, the diameter of the periphery 7 of the coating layer 6 is enlarged. The coating layer 6 is formed of a polyolefine resin such as polyethylene or rubber, and the fiber bundle 3 may be coated by inserting the same into the heat shrinkable tube.

Fig. 6 is a vertical sectional view showing one embodiment of the present invention during use. Referring to Fig. 6, the light emitted from the tip end surface of the light transmitting optical fiber 1 is condensed by the lens 4 and enter the living tissue 5 as shown by the arrow in Fig. 6. The incident light is scattered and reflected to enter the light receiving optical fiber 2 again through the lens 4. On this occasion, the substantial distance between the light transmitting optical fiber 1 and the light receiving optical fiber 2 is ℓ' in Fig. 6 and the depth of the light reaching the living tissue is d'. The depth d' is shallower than the conventional measuring probe, so that the measuring probe of the present invention is capable of measuring organs containing much blood.

The lens 4 employed in this embodiment should preferably be planar as shown in Fig. 6 in order to enable close contact with the living tissue 5 and the surface of the lens to be in contact with the living tissue 5 and to prevent fluctuation of the measured value caused by vibration of the probe. In addition, in order to realize uniform contact and to reduce optical loss, the contact surface of the lens 4 with the light illuminating fiber 1 and the light receiving fiber 2 should be planar. An example of such lens is a selfoc lens.

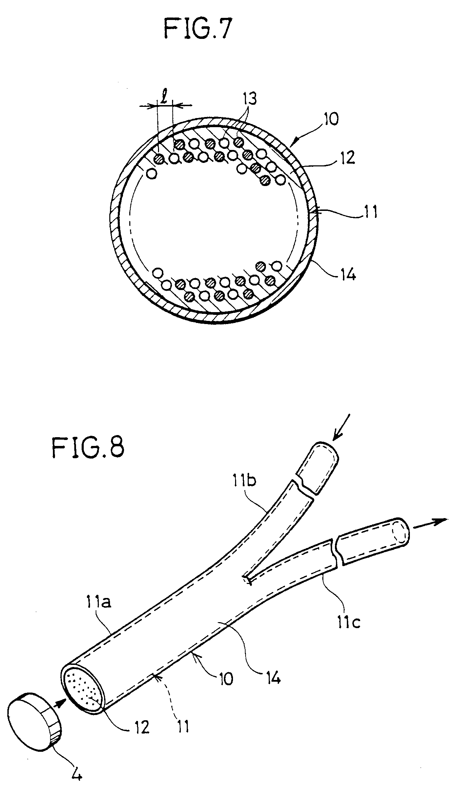

Fig. 7 is a cross section showing an end portion of the probe without the condensing means. Fig. 8 is a perspective view showing the whole structure.

Referring to Fig. 7, the measuring probe 10 comprises an image fiber 11. The image fiber 11 comprises a number of cores 13 each having a diameter of several to several 10 »m arranged in parallel to be a matrix in the clad 12 such that the thickness of the clad in the core 13 is several »m. At the tip end portion 11a of the probe, one of the adjacent cores is used for illuminating light and the other one is used for receiving light. In Fig. 7, cores for illuminating light are shown white and the cores for receiving light are hatched so as to facilitate understanding.

These cores 13 are divided into groups, that is, the light transmitting cores and the light receiving cores, and branched at an intermediate portion of the measuring probe 10. The branched cores are respectively bundled covered by clads or integrally arranged in a matrix in the clad, providing branched image fibers 11b and 11c. The branched image fiber 11b is connected to a light source of an analyzing apparatus and the branched image fiber 11c is connected to a spectrometer of the apparatus. The outer periphery of the image fiber 11 is covered by a light shading flexible coating 14 formed of a non-transparent material. In this embodiment, a black polyvinyl chloride resin is used as the coating 14. A holder may be provided on the outermost periphery of the probe tip end 11a so as to protect the flow and to facilitate handling of the measuring probe 10.

Assuming that the diameter of the core 13 is 30 »m in the measuring probe structured as described above, the distance ℓ1 between the centers of the light illuminating core and the light receiving core, which is represented by a sum of the core diameter and the thickness of the clad between cores, can be made 40 »m or less, while the distance in the conventional probe was about 100 »m. Therefore, the value of the depth D described with reference to Fig. 1 is made no more than one half of the conventional probe. Therefore, naturally, the sample area moves to the portion in the left than the portion b in Fig. 3, namely, the area at which the optical density changes linearly, free from the influence of the scattering effect.

A condensing means such as the selfoc lens 4 is provided at the end surface of the image fiber 11.

## Claims

1. A fiber optic probe for measuring reflectance spectrum for emitting light to a surface of a living body and for analyzing bY spectrophotometry, light transmitted and reflected from the living body, comprising:
a fiber bundle (3; 12, 13) having a light illuminating optical fiber for illuminating light and a light receiving optical fiber for receiving light transmitted and reflected from said living body arranged in parallel; and
condenser means (4) provided over end surfaces of said light illuminating optical fiber and said light receiving optical fiber,
**characterized by**
a coating layer (6, 14) formed of a heat shrinkable tube for fixing said condenser means (4) on said light illuminating optical fiber and said light receiving optical fiber by covering periphery of said fiber bundle (3; 12, 13) and by covering, with end portion thereof, periphery of said condenser means.

2. A fiber optic probe for measuring reflectance spectrum according to claim 1, wherein said condenser means (4) comprises a lens having a surface which is to be in contact with a living body made flat and a flat surface which is to be in contact with said fiber bundle.

3. A fiber optic probe for measuring reflectance spectrum according to claim 1 or 2, wherein said condenser means (4) is provided detachably.

4. A fiber optic probe for measuring reflectance spectrum according to any of the preceding claims, wherein said fiber bundle (12, 13) comprises a clad; and
a plurality of light illuminating optical fiber cores and a plurality of light receiving optical fiber cores arranged adjacent to each other in a matrix in said clad (12).

## Patentansprüche

1. Fiberoptische Sonde zum Messen des Reflexionsspektrums zum Emittieren von Licht zu einer Oberfläche eines lebenden Körpers und zum spektro-photometrischen Analysieren des Lichtes, das durch den lebenden Körper übertragen und von dem lebenden Körper reflektiert wurde, umfassend:
ein Fiberbündel (3; 12, 13) mit einer optischen Lichtbeleuchtungsfiber für Beleuchtungslicht und einer optischen Lichtempfangsfiber zum Empfangen von Licht, das durch den lebenden Körper transmittiert und reflektiert wurde, in paralleler Anordnung; und
eine Kondensoreinrichtung (4), welche über Endflächen der optischen Beleuchtungsfiber bzw. der optischen Lichtempfangsfiber vorgesehen sind,
**gekennzeichnet durch**
eine Überzugsschicht (6, 14), welche durch eine wärmeschrumpfende Röhre zum Befestigen der Kondensoreinrichtung (4) an der optischen Beleuchtungsfiber und der optischen Empfangsfiber durch Bedecken des Umfangs des Fiberbündels (3; 12,13) und durch Bedecken des Umfangs der Kondensoreinrichtung mit dem Endteil desselben.

2. Fiberoptische Sonde zum Messen des Reflexionsspektrums nach Anspruch 1, worin die Kondensoreinrichtung (4) einer Linse umfaßt, welche eine Oberfläche aufweist, die in Kontakt mit einem lebenden Körper stehen soll und die abgeflacht ist, und eine flache Oberfläche, welche in Kontakt mit dem Fiberbündel steht.

3. Fiberoptische Sonde zum Messen des Reflexionsspektrums nach Anspruch 1 oder 2, worin die Kondensoreinrichtung (4) in abnehmbarer Weise vorgesehen ist.

4. Fiberoptische Sonde zum Messen des Reflexionsspektrums nach einem der vorangegangenen Ansprüche, worin das Fiberbündel (12,13) einen Überzug umfaßt; und
eine Anzahl von optischen Beleuchtungsfiberkerne und eine Anzahl von optischen Empfangsfiberkerne nebeneinander in einer Matrix in dem Überzug (12) angeordnet sind.

## Revendications

1. Sonde à fibres optiques pour mesurer le spectre de réflectance, destinée à émettre de la lumière sur une surface d'un corps vivant pour analyser par spectrophotométrie la lumière transmise et réfléchie par le corps vivant, comprenant:
un premier faisceau (3,12,13) ayant une fibre optique d'éclairage lumineux destinée à produire un éclairage lumineux et une fibre optique réceptrice de lumière pour recevoir la lumière transmise et réfléchie par le corps vivant, disposées en parallèle et
un condensateur monté sur les surfaces d'extrémité de la fibre optique d'éclairage lumineux et de la fibre optique réceptrice de lumière,
caractérisée par une couche de revêtement (6,14) constituée par un tube thermorétractable pour fixer le condensateur (4) sur la fibre optique d'éclairage lumineux et sur la fibre optique réceptrice de lumière en recouvrant la périphérie du faisceau de fibres (3,12,13) et en recouvrant, par sa portion d'extrémité, la périphérie du condensateur.

2. Sonde à fibres optiques, destinée à mesurer le spectre de réflectance selon la revendication 1, dans laquelle le condensateur (4) comprend une lentille avec une surface qui doit être en contact avec un corps vivant mis à plat et une surface plane qui doit être en contact avec le faisceau de fibres.

3. Sonde à fibres optiques, destinée à mesurer le spectre de réflectance selon la revendication 1 ou 2, dans laquelle le condensateur (4) est monté de façon amovible.

4. Sonde à fibres optiques, destinée à mesurer le spectre de réflectance selon l'une quelconque des revendications précédentes, dans laquelle le faisceau de fibres (12,13) comprend un habillage; et
une pluralité de noyaux de fibres optiques d'éclairage lumineux et une pluralité de noyaux de fibres optiques recevant la lumière, disposés de façon contiguë entre eux dans une matrice intérieure de l'habillage (12).
